(19) Europäisches Patentamt
European Patent Office
Office européen des brevets

(11)  EP 0 841 319 B1

(12)  **EUROPEAN PATENT SPECIFICATION**

(45) Date of publication and mention
of the grant of the patent:
**20.06.2001   Bulletin 2001/25**

(51) Int Cl.[7]: **C07C 67/54**, C07C 67/20

(21) Application number: **97118881.8**

(22) Date of filing: **30.10.1997**

(54) **Process for preparing lactate**

Verfahren zur Herstellung von Milchsäureestern

Procédé de préparation de lactates

(84) Designated Contracting States:
**DE ES FR GB NL**

(30) Priority: **07.11.1996   JP 29520396**

(43) Date of publication of application:
**13.05.1998   Bulletin 1998/20**

(73) Proprietor: **MITSUBISHI GAS CHEMICAL
COMPANY, INC.
Chiyoda-ku, Tokyo (JP)**

(72) Inventors:
• **Abe, Takafumi, Mitsubishi Gas Chem. Comp. Inc.
Tayuhama, Niigata-shi, Niigata-ken (JP)**

• **Shima, Yoshikazu,
Mitsubishi Gas Chem. Comp. Inc.
Tayuhama, Niigata-shi, Niigata-ken (JP)**
• **Ikemoto, Kazuto,
Mitsubishi Gas Chem. Comp. Inc.
Tayuhama, Niigata-shi, Niigata-ken (JP)**

(74) Representative:
**Gille Hrabal Struck Neidlein Prop Roos
Patentanwälte
Brucknerstrasse 20
40593 Düsseldorf (DE)**

(56) References cited:
**EP-A- 0 672 645          US-A- 4 159 925
US-A- 4 975 157**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

1. Field of the Invention

The present invention relates to a process for preparing a lactate, and more specifically, it relates to a novel process for preparing a lactate from acetaldehyde and a formate which are starting materials. The lactates have been used in large quantities as solvents for coating materials, as solvents for use in the electronic industry, and as materials for organic synthesis of medicines and the like. Therefore, the lactates are industrially extremely important chemicals.

2. Description of the Related Art

An industrial method for preparing a lactate usually comprises synthesizing cyanohydrin from prussic acid and acetaldehyde as starting materials, hydrolyzing the cyanohydrin, and then esterifying. However, this method has a drawback that a large amount of ammonium salts is secondarily produced and its treatment leads to the increase in manufacturing cost of the lactate.

As the other techniques for preparing lactic acid, there are known a method which comprises allowing dinitrogen tetroxide to act on a terminal olefin, followed by hydrolysis; a method which comprises reacting acetaldehyde with carbon monoxide and water in the presence of a noble metal catalyst or an acidic catalyst; and a method which comprises halogenating the α-position of a carboxylic acid, followed by hydrolysis. However, these methods are poor in yields, utilize limited material sources, require troublesome operations, and also require expensive catalysts. For these reasons, the above-mentioned methods are ineligible for industrial methods for preparing lactic acid on a large scale. In fact, these methods have been used to specifically produce lactic acid and its derivatives only on a small scale.

Therefore, the present inventors have previously found a process for preparing a lactate via a cyanohydrin without forming by-products of the ammonium salts (Japanese Patent Application Laid-open No. 233122/1995). The technique described in this Japanese Patent Application Laid-open No. 233122/1995 is a method for preparing the lactate and formamide from lactamide and a formate, but when components having lower boiling points than the lactate are recovered from this amide-ester exchange reaction solution by distillation and if this recovery is done under a pressure of atmospheric pressure or more, a bottom temperature of distillation column rises, so that 2-formyloxy propionate (the formate of the lactate) is produced, which results in the deterioration of the yield of the lactate. In addition, since the boiling point of this 2-formyloxy propionate is close to that of the lactate, the expensive distillation column having high separation efficiency is required in order to accomplish the separation by the distillation. If the separation by the distillation is done under reduced pressure in order to avoid the above-mentioned inconvenience, there is a drawback that expensive refrigeration facilities are necessary in order to recover low-boiling components such as the formate.

SUMMARY OF THE INVENTION

The present invention has been developed to overcome the above-mentioned drawbacks, and an object of the present invention is to provide a process for preparing a lactate in a high yield, the production of the 2-formyloxy propionate being restrained. The production route of this 2-formyloxy propionate is not definite, but it can be presumed that the production is made by the following reaction:

$$CH_3CH(OH)COOR^1 + HCOOR^2 \rightarrow HCOOCH(CH_3)COOR^1 + R^2OH$$

(R[1] and R[2] are each an alkyl group), or

$$CH_3CH(OH)COOR^1 + HCONH_2 \rightarrow HCOOCH(CH_3)COOR^1 + NH_3$$

(R[1] and R[2] are each an alkyl group)

Furthermore, according to the present invention, the production of a dimer of the lactate (an alkyllactoyl lactate) can also be restrained by lowering a bottom temperature of the distillation column, which can prevent the yield of the lactate from deteriorating.

That is to say, the present invention is directed to a process for preparing a lactate represented by the general formula $CH_3CH(OH)COOR$ (R is an alkyl group having 1 to 8 carbon atoms) by the reaction of lactamide and a formate which comprises the step of recovering components having lower boiling points than the lactate from a reaction solution by distillation under a condition that M is 16 or less, M being defined as follows: Retention time of liquid in the bottom of the distillation column (hour) multiplied by (the temperature of the bottom of the distillation column minus 126) (°C). Furthermore, in the present invention, the recovery of the components having lower boiling points than the lactate can be carried out more economically and effectively by the distillation under a pressure of atmospheric pressure or more.

## DESCRIPTION OF THE PREFERRED EMBODIMENTS

Lowering a bottom temperature of distillation column under a pressure of atmospheric pressure or more can be achieved usually by adding a solvent having a low boiling point so as to increase the concentration of this solvent in a column bottom liquid until a desirable bottom temperature has been reached. Any solvent can be used, so long as it has a low boiling point, but it is preferably a solvent which can be used in the reaction of lactamide and a formate, more preferably an alcohol corresponding to the formate which can be used during an amide ester exchange reaction.

A temperature level to which the temperature of column bottom should be lowered depends on a retention time of the liquid on the column bottom, and generally speaking, the longer the retention time is, the lower the allowable temperature level is. According to the results variously investigated by the present inventors, it has been found that when an M value represented by the following equation is 16 or less, the successful results can be obtained:

$$M = [\text{retention time}] \text{ (hour)} \times [\text{temperature of column bottom} - 126] \text{ (°C)}$$

According to the present invention, the desired lactate can be purified by the distillation even under a pressure of atmospheric pressure or more, while the production of the 2-formyloxy propionate is restrained, so that the lactate can be prepared in a high yield.

Next, the present invention will be described in more detail in accordance with examples, but the scope of the present invention should not be limited to these examples.

## Example 1

A stainless steel reaction tube (inner diameter = 15 mm x length = 350 mm) was filled with 50 ml of a strongly basic anion exchange resin (Amberlite 900, made by Rhom & Harth Co., Ltd.) which was previously converted into an OH type by a treatment with an aqueous 1N NaOH solution, and warm water was then circulated through a jacket so as to maintain the temperature of an anion exchange resin layer at 50°C. A mixed solution of lactamide, methyl formate and methanol (a molar ratio = 1:2:3) was fed to this layer at 16.6 g/hr. After 200 hours had lapsed from the start of reaction, the resulting reaction solution was sampled for 1 hour, and then analyzed by a gas chromatography. As a result, a conversion of lactamide was 60.9%, and the selectivities of methyl lactate and formamide on the basis of lactamide to be reacted were 99.2% and 99.0%, respectively.

Next, the distillation under atmospheric pressure was carried out by the use of the reaction solution obtained in this example.

This reaction solution was fed at 200 g/hr to a middle stage of a distillation tower, which consists of 2 columns in series (each having a 30 mm inner diameter and 300 mm length)filled with McMahons packing of 6 mm size and having a jacketed container with inner volume of about 50 ml at the bottom thereof. While methyl formate and methanol were distilled off from a tower top at a reflux ratio of 0.5 and a tower bottom liquid containing methyl lactate was drawn from the column bottom, the column bottom container was heated so that a column bottom temperature might be kept constant. The change of concentrations of methyl-2-formyloxy propionate in the tower bottom liquid at the various column bottom temperatures is shown in Table 1.

Table 1

| Methanol (wt%)[1] | Column Bottom Temp. (°C) | Average Retention Time (hr) | Methyl 2-formyloxy Propionate (wt%)[2] | Molar Yield of Methyl 2-formyloxy Propionate (%)[3] | M Value |
|---|---|---|---|---|---|
| 5.0 | 135 | 0.58 | 0.03 | 0.06 | 5.2 |
| 2.6 | 152 | 0.59 | 0.03 | 0.06 | 15.3 |
| 2.0 | 158 | 0.59 | 0.78 | 1.59 | 18.9 |
| Trace | 165 | 0.60 | 1.71 | 3.42 | 23.4 |

1) A concentration of methanol in the tower bottom liquid.

2) The concentration of methyl 2-formyloxy propionate in the tower bottom liquid.

3) A molar yield of 2-formyloxy propionate based on the methyl lactate fed to the distillation column.

Example 2

Distillation was carried out by the same procedure as in Example 1 except that a feed rate of reaction solution to the distillation column was 100 g/hr. The results are shown in Table 2.

4

Table 2

| Methanol (wt%)[1] | Column Bottom Temp. (°C) | Average Retention Time (hr) | Methyl 2-formyloxy Propionate (wt%)[2] | Molar Yield of Methyl 2-formyloxy Propionate (%)[3] | M Value |
|---|---|---|---|---|---|
| 9.1 | 121 | 1.1 | 0.02 | 0.04 | -5.5 |
| 5.0 | 135 | 1.2 | 0.03 | 0.06 | 10.8 |
| 3.5 | 148 | 1.2 | 1.35 | 2.70 | 26.4 |
| 2.0 | 158 | 1.2 | 2.04 | 4.16 | 38.4 |

1) A concentration of methanol in the tower bottom liquid.

2) The concentration of methyl 2-formyloxy propionate in the tower bottom liquid.

3) A molar yield of 2-formyloxy propionate based on the methyl lactate fed to the distillation column.

**Claims**

1. A process for preparing a lactate represented by the general formula $CH_3CH(OH)COOR$ (R is an alkyl group having 1 to 8 carbon atoms) by the reaction of lactamide and a formate which comprises the step of recovering components having lower boiling points than the lactate from a reaction solution by distillation under a condition that M is 16 or less, M being defined as follows: M = [Retention time] (hour) x [temperature of column bottom - 126] (°C).

2. The process for preparing a lactate according to Claim 1 wherein the components having lower boiling points than the lactate are recovered by distillation under atmospheric pressure or more.

**Patentansprüche**

1.  Verfahren zur Herstellung eines durch die allgemeine Formel $CH_3CH(OH)COOR$ (R ist eine Alkylgruppe mit 1 bis 8 Kohlenstoffatomen) dargestellten Lactats durch Reaktion von Lactamid mit einem Formiat, welches die Stufen Isolierung von Komponenten mit niedrigeren Siedepunkten als der des Lactats aus einer Reaktionslösung durch Destillation unter der Bedingung umfaßt, dass M 16 oder kleiner ist, wobei M wie folgt definiert ist: M = [Retentionszeit] (Stunde) x [Temperatur des Kolonnenbodens - 126] (°C).

2.  Verfahren zur Herstellung eines Lactats nach Anspruch 1, wobei die Komponenten mit niedrigeren Siedepunkten als der des Lactats durch Destillation unter Atmosphärendruck oder mehr isoliert werden.


**Revendications**

1.  Procédé pour préparer un lactate représenté par la formule générale $CH_3CH(OH)COOR$ (R est un groupe alkyle possédant 1 à 8 atomes de carbone) consistant à faire réagir un lactamide et un formiate qui comprend l'étape consistant à récupérer des composants possédant des points d'ébullition inférieurs à celui du lactate à partir d'une solution réactionnelle, par distillation, à condition que M vaille 16 ou moins, M étant défini comme il suit :

    $$M = [\text{temps de rétention] (heure)} \times [\text{température du fond de la colonne - 126] } (°C).$$

2.  Procédé pour préparer un lactate selon la revendication 1, dans lequel les composants possédant des points d'ébullition inférieurs au lactate sont récupérés par distillation sous une pression égale ou supérieure à la pression atmosphérique.